# EUROPEAN PATENT SPECIFICATION

(11) **EP 1 654 003 B1**
(45) Date of publication and mention of the grant of the patent: **19.12.2007**
(21) Application number: 04817748.9
(22) Date of filing: 11.08.2004
(51) Int. Cl.: A61K 36/746

(54) **PREVENTATIVE EFFECTS OF MORINDA CITRIFOLIA ON MAMMARY BREAST CANCER**
PRÄVENTIVE WIRKUNGEN VON MORINDA CITRIFOLIA AUF BRUSTKREBS
EFFETS PREVENTIFS DE MORINDA CITRIFOLIA SUR LE CANCER DU SEIN

(30) Priority: 12.08.2003 US 639833
(43) Date of publication of application: 10.05.2006
(73) Proprietor: Tahitian Noni International, Inc, Provo, UT 84604 (US)
(72) Inventor: WANG, Mian-Ying, Rockford, IL 61107 (US); JENSEN, Claude, Jarakae, Cedar Hills, UT 84062 (US); SU, Chen, West Jordan, UT 84084 (US)
(74) Representative: Bone, Alexander Marcus Thomas
(86) International application number: PCT/US2004/026253
(87) International publication number: WO 2005/048919

(56) References cited:
- WO-A2-02/45654
- FR-A1- 2 783 137
- JP-A- 6 087 736
- WANG M-Y ET AL: "MORINDA CITRIFOLIA (NONI): A LITERATURE REVIEW AND RECENT ADVANCES IN NONI RESEARCH" ACTA PHARMACOLOGICA SINICA, CN, vol. 23, no. 12, 23 December 2002 (2002-12-23), pages 1127-1141, XP008016134 ISSN: 1671-4083
- WANG M Y ET AL: "CANCER PREVENTIVE EFFECT OF MORINDA CITRIFOLIA (NONI)" ANNALS OF THE NEW YORK ACADEMY OF SCIENCES, NEW YORK ACADEMY OF SCIENCES, NEW YORK, NY, US, vol. 952, December 2001 (2001-12), pages 161-168, XP008016136 ISSN: 0077-8923

## Description

### Field of the Invention

The present invention relates to medicinal products, as well as to health and wellness food products, and particularly to a medicinal product or a health and wellness food product designed to inhibit, block, and prevent further growth of carcinogenic cells, as well as to destroy existing carcinogenic cells, within the mammary region of the breast. Stated differently, the present invention relates to inhibiting, blocking, and preventative methods of treatment for mammary breast cancer.

### Background of the Invention and Related Art

Thousands of Americans are diagnosed with breast cancer each year. In the U.S. in 2002, it was estimated that more than 255,000 women and 1500 men were diagnosed and nearly 40,000 women and 400 men died from the disease. Treatment protocols for breast cancer are much the same as those for other types of cancer. Such methods include surgery, radiation therapy, chemotherapy and hormone therapy.

Whereas surgery is often used as a last resort, radiation therapy, chemotherapy and/or hormone therapy may be implemented at any stage of breast cancer to inhibit and/or destroy malignant tumor growth. Although such cytotoxic treatments are often highly successful, such treatments also destroy substantial numbers of healthy cells and, particularly in the case of hormone therapy, may actually increase a patient's chances of developing other types of cancer. Some of the side effects typically experienced by those undergoing traditional breast cancer treatment include nausea, diarrhea, hair loss, and hypersensitivity to light, and weight loss. These debilitating side effects limit the frequency and dosage at which such treatments may be administered, thereby limiting the perceived effect of the treatment and requiring longer periods of such treatment over time.

Accordingly, what is needed is a composition for inhibiting tumor growth in breast cancer patients that does not cause ancillary debilitating sickness. What is also needed is a composition for inhibiting mammary tumor growth that limits ancillary weight loss. Finally what is needed is a compound having an anti-tumorigenesis effect that may be aggressively administered over a relatively short period of time to effectively inhibit and/or destroy tumor growth within that period of time without causing harmful side effects.

Such compounds are claimed herein.

### BACKGROUND ART

European Patent Application No. 02252679.2 which has been published as EP 1250849 A1 discloses the use of Morinda Citrifolia at a concentration of 10-30% by weight and MSM (Methyl Sulfonyl Methane) at a concentration of <1% by weight in an animal food formulation intended to provide improved muscle and bone support.

### SUMMARY OF THE INVENTION

The present invention teaches compounds containing an effective amount of *Morinda citrifolia* fruit juice to inhibit the conversion of mammary cells to tumors. The present invention provides a non-toxic compound having an anti-tumorigenesis effect, wherein the non-toxic compound comprises an effective amount of *Morinda citrifolia* product selected from *Morinda citrifolia* fruit juice and methyl sulfonyl methane and water wherein the Morinda citrifolia fruit juice is present in a amount of 5 percent by weight and wherein the methyl sulfonyl methane is present in an amount of 5 percent by weight.

The present invention provides a composition for inhibiting tumor growth in breast cancer patients that does not cause ancillary debilitating sickness.

The present invention provides a composition for inhibiting mammary tumor growth that limits ancillary weight loss.

Furthermore, the present invention provides a compound having an anti-tumorigenesis effect that may be aggressively administered over a relatively short period of time to effectively inhibit and/or destroy tumor growth within that period of time without causing harmful side effects.

### BRIEF DESCRIPTION OF THE DRAWINGS

The invention will now be described by way of example only with reference to the accompanying drawings in which:
Figure 1 is a graphical representation of the effectiveness of certain *Morinda* citrifolia-containing compounds on the prevalence of tumors in accordance with the present invention;
Figure 2 is a graphical representation of the preventive effects of *Morinda* citrifolia-containing compounds on mammary gland tumorigenesis induced by estrogen in female ACI rats in accordance with the present invention;
Figure 3 is a graphical representation of the relative body weight of rats that have been implanted with estrogen to induce tumorigenesis, wherein certain rats have been treated with *Morinda citrifolia*-containing compounds to counteract the effects estrogen-induced tumorigenesis in accordance with the present invention;
Figure 4 is a graphical representation of the relative size of tumors in rats treated with various compounds; and

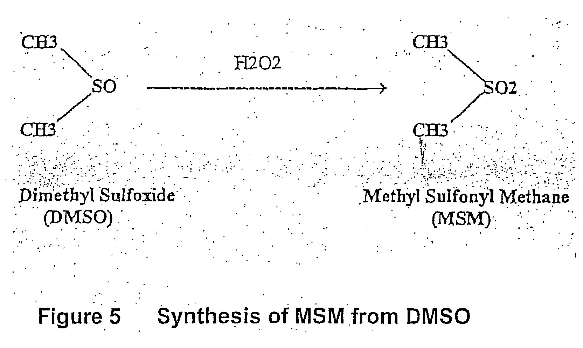

Figure 5 is a graphical representation of the conversion of Dimethyl Sulfoxide to Methyl Sulfonyl Methane in accordance with certain embodiments of the present invention.

### DETAILED DESCRIPTION OF THE PREFERRED EMBODIMENTS

The present invention describes compostions for treating breast cancer, and particularly the inhibition, blocking, and/or prevention of cancerous cell growth within the mammary region of the breast, as well as a formulation for destroying existing cancerous cells within the breast using a formulation comprising *Morinda citrifolia* in processed form.

The following detailed description is separated into sections to more clearly point out and present the advantages and aspects of the present invention. A general description of *Morinda citrifolia,* including its origins, processing techniques, and health benefits is explained below, followed by a more detailed description of the *Morinda citrifolia*-based formulations and compositions used to treat breast cancer, including examples of experimental studies and the results attained.

### GENERAL DESCRIPTION OF MORINDA CITRIFOLIA

The Indian Mulberry or Noni plant, known scientifically as Morinda citrifolia L. *(Morinda citrifolia),* is a shrub or small tree up to 10 m in height. The leaves are oppositely arranged with an elliptic to ovate form. The small white flowers are contained in a fleshy, globose, head-like cluster. The fruits are large, fleshy, and ovoid. At maturity, they are creamy-white and edible, but have an unpleasant taste and odor. The plant is native to Southeast Asia and has spread in early times to a vast area from India to eastern Polynesia. It grows randomly in the wild, and it has been cultivated in plantations and small individual growing plots. The *Morinda citrifolia* flowers are small, white, three to five lobed, tubular, fragrant, and about 1.25 cm long. The flowers develop into compound fruits composed of many small drupes fused into an ovoid, ellipsoid or roundish, lumpy body, with waxy, white, or greenish-white or yellowish, semi-translucent skin. The fruit contains "eyes" on its surface, similar to a potato. The fruit is juicy, bitter, dull-yellow or yellowish-white, and contains numerous red-brown, hard, oblong-triangular, winged 2-celled stones, each containing four seeds.

When fully ripe, the fruit has a pronounced odor like rancid cheese. Although the fruit has been eaten by several nationalities as food, the most common use of the *Morinda citrifolia* plant was as a red and yellow dye source. Recently, there has been an interest in the nutritional and health benefits of the *Morinda citrifolia* plant, further discussed below.

Because the *Morinda citrifolia* fruit is for all practical purposes inedible, the fruit must be processed in order to make it palatable for human consumption and included in food products used to treat Candidiasis. Processed *Morinda citrifolia* fruit juice can be prepared by separating seeds and peels from the juice and pulp of a ripened *Morinda citrifolia* fruit; filtering the pulp from the juice; and packaging the juice. Alternatively, rather than packaging the juice, the juice can be immediately included as an ingredient in another food product, frozen or pasteurized. In some embodiments, the juice and pulp can be pureed into a homogenous blend to be mixed with other ingredients. Other process include freeze drying the fruit and juice. The fruit and juice can be reconstituted during production of the final juice product. Still other processes include air drying the fruit and juices, prior to being masticated.

The present invention utilizes the fruit juice and the oil extracted from the *Morinda citrifolia* plant. In a currently preferred process of producing *Morinda citrifolia* fruit juice, the fruit is either hand picked or picked by mechanical equipment. The fruit can be harvested when it is at least one inch (2-3 cm) and up to 12 inches (24-36 cm) in diameter. The fruit preferably has a color ranging from a dark green through a yellow-green up to a white color, and gradations of color in between. The fruit is thoroughly cleaned after harvesting and before any processing occurs.

The fruit is allowed to ripen or age from 0 to 14 days, with most fruit being held from 2 to 3 days. The fruit is ripened or aged by being placed on equipment so it does not contact the ground. It is preferably covered with a cloth or netting material during aging, but can be aged without being covered. When ready for further processing the fruit is light in color, from a light green, light yellow, white or translucent color. The fruit is inspected for spoilage or for excessively green color and hard firmness. Spoiled and hard green fruit is separated from the acceptable fruit.

The ripened and aged fruit is preferably placed in plastic lined containers for further processing and transport. The containers of aged fruit can be held from 0 to 30 days. Most fruit containers are held for 7 to 14 days before processing. The containers can optionally be stored under refrigerated conditions prior to further processing. The fruit is unpacked from the storage containers and is processed through a manual or mechanical separator. The seeds and peel are separated from the juice and pulp.

The juice and pulp can be packaged into containers for storage and transport. Alternatively, the juice and pulp can be immediately processed into finished juice product. The containers can be stored in refrigerated, frozen, or room temperature conditions. The *Morinda citrifolia* juice and puree are preferably blended in a homogenous blend, after which they may be mixed with other ingredients, such as flavorings, sweeteners, nutritional ingredients, botanicals, and colorings. The finished juice product is preferably heated and pasteurized at a minimum temperature of 181°F (83 °C) or higher up to 212°F (100°C).

The product is filled and sealed into a final container of plastic, glass, or another suitable material that can withstand the processing temperatures. The containers are maintained at the filling temperature or may be cooled rapidly and then placed in a shipping container. The shipping containers are preferably wrapped with a material and in a manner to maintain or control the temperature of the product in the final containers.

The juice and pulp may be further processed by separating the pulp from the juice through filtering equipment. The filtering equipment preferably consists of a centrifuge decanter, a screen filter with a size from 1 micron up to 2000 microns, more preferably less than 500 microns, a filter press, reverse osmosis filtration, and any other standard commercial filtration devices. The operating filter pressure preferably ranges from 0.1 psig up to about 1000 psig. The flow rate preferably ranges from 0.1 g.p.m. up to 1000 g.p.m., and more preferably between 5 and 50 g.p.m. The wet pulp is washed and filtered at least once and up to 10 times to remove any juice from the pulp. The wet pulp typically has a fiber content of 10 to 40 percent by weight. The wet pulp is preferably pasteurized at a temperature of 181 °F (83°C) minimum and then packed in drums for further processing or made into a high fiber product.

The method for extracting and processing the oil is described in co-pending Application Serial No. 09/384,785, filed on August 27, 1999.

The *Morinda citrifolia* oil typically includes a mixture of several different fatty acids as triglycerides, such as palmitic, stearic, oleic, and linoleic fatty acids, and other fatty acids present in lesser quantities. In addition, the oil preferably includes an antioxidant to inhibit spoilage of the oil. Conventional food grade antioxidants are preferably used.

The *Morinda citrifolia* plant is rich in natural ingredients. Those ingredients that have been discovered include: from the leaves: alanine, anthraquinones, arginine, ascorbic acid, aspartic acid, calcium, beta-carotene, cysteine, cystine, glycine, glutamic acid, glycosides, histidine, iron, leucine, isoleucine, methionine, niacin, phenylalanine, phosphorus, proline, resins, riboflavin, serine, beta-sitosterol, thiamine, threonine, tryptophan, tyrosine, ursolic acid, and valine; from the flowers: acacetin-7-o-beta-d(+)-glucopyranoside,
5,7-dimethyl-apigenin-4'-o-beta-d(+)-galactopyranoside, and
6,8-dimethoxy-3-methylanthraquinone-1-o-beta-rhamnosyl-glucopyranoside; from the fruit: acetic acid, asperuloside, butanoic acid, benzoic acid, benzyl alcohol,
1-butanol, caprylic acid, decanoic acid, (E)-6-dodeceno-gamma-lactone, (Z,Z,Z)-8,11,14-eicosatrienoic acid, elaidic acid, ethyl decanoate, ethyl hexanoate, ethyl octanoate, ethyl palmitate, (Z)-6-(ethylthiomethyl) benzene, eugenol, glucose, heptanoic acid, 2-heptanone, hexanal, hexanamide, hexanedioic acid, hexanoic acid (hexoic acid), 1-hexanol, 3-hydroxy-2-butanone, lauric acid, limonene, linoleic acid, 2-methylbutanoic acid, 3-methyl-2-buten-1-ol, 3-methyl-3-buten-1-ol, methyl decanoate, methyl elaidate, methyl hexanoate, methyl 3-methylthio-propanoate, methyl octanoate, methyl oleate, methyl palmitate, 2-methylpropanoic acid, 3-methylthiopropanoic acid, myristic acid, nonanoic acid, octanoic acid (octoic acid), oleic acid, palmitic acid, potassium, scopoletin, undecanoic acid,
(Z,Z)-2,5-undecadien-1-ol, and vomifol; from the roots: anthraquinones, asperuloside (rubichloric acid), damnacanthal, glycosides, morindadiol, morindine, morindone, mucilaginous matter, nor-damnacanthal, rubiadin, rubiadin monomethyl ether, resins, soranjidiol, sterols, and trihydroxymethyl anthraquinone-monomethyl ether; from the root bark: alizarin, chlororubin, glycosides (pentose, hexose), morindadiol, morindanigrine, morindine, morindone, resinous matter, rubiadin monomethyl ether, and soranjidiol; from the wood: anthragallol-2,3-dimethylether; from the tissue culture: damnacanthal, lucidin, lucidin-3-primeveroside, and
morindone-6beta-primeveroside; from the plant: alizarin, alizarin-alpha-methyl ether, anthraquinones, asperuloside, hexanoic acid, morindadiol, morindone, morindogenin, octanoic acid, and ursolic acid.

Reference compositions or formulations and their uses:

| | Formulation One | |
|---|---|---|
| Ingredients | | Percent by Weight |
| | | |
| *Morinda citrifolia* Fruit Juice | | 100% |
| | Formulation Two | |
| Ingredients | | Percent by Weight |
| *Morinda citrifolia* Fruit Juice | | 85-99.99% |
| Water | | 0.1-15% |
| | Formulation Three | |
| Ingredients | | Percent by Weight |
| *Morinda citrifolia* Fruit Juice | | 85-99.99% |
| Other fruit juices | | 0.1-15% |
| | Formulation Four | |
| | | |
| Ingredients | | Percent by Weight |
| *Morinda citrifolia* Fruit Juice | | 50-90% |
| Water | | 0.1-50% |
| Other fruit juices | | 0.1-30% |

In one use, a person suffering from mammary breast cancer as described above takes at least one (1) ounce (29.57 cm³) of Formulation One in the morning on an empty stomach, and at least one (1) ounce (29.57 cm³) at night on an empty stomach, just prior to retiring to bed. In one example the beneficial *Morinda Citrifolia* is processed into Tahitian Noni® juice manufactured by Morinda, Incorporated of Orem, Utah.

In another use, a person diagnosed with or experiencing symptoms of breast cancer takes at least one ounce (29.57 cm³) of Formulation Two twice a day until the overgrowth is abated.

### Example One

In the present example, a patient experiencing or diagnosed with and is suffering from mammary breast cancer desires to treat the condition with a nonprescription, over-the-counter preparation. To treat the cancer, the individual consumes an identified prescribed amount of a food product composition containing processed *Morinda citrifolia* fruit juice. The person intermittently consumes the food product containing the processed *Morinda citrifolia* fruit juice until the carcinogenic cells within the mammary are inhibited, blocked, and/or destroyed, wherein the infection is reduced or eliminated.

### Example Two: Effect of Morinda citrifolia-containing compounds on E2-induced mammary tumors

Sixty five-week old female rats were divided into four groups of fifteen rats each and placed on regular diets. Another eight female rats served as age-matched controls. One group of experimental rats was given 5% placebo in drinking water, the second experimental group was given 5% Morinda citrifolia juice in drinking water, the third experimental group was given 5% methyl sulfonyl methane ("MSM") in drinking water, and the fourth experimental group was given a combination of 5% Morinda citrifolia juice and 5% MSM in drinking water. Two weeks later, all animals were implanted subcutaneously with a 25 mg pellet containing 22.5 mg of 17β-estradiol (E2) mixed with 2.5 mg cholesterol. Each experimental group was provided with its respective formulation for ninety days following estrogen (E2) implantation. The age-matched control animals received a 25 mg cholesterol pellet implant.

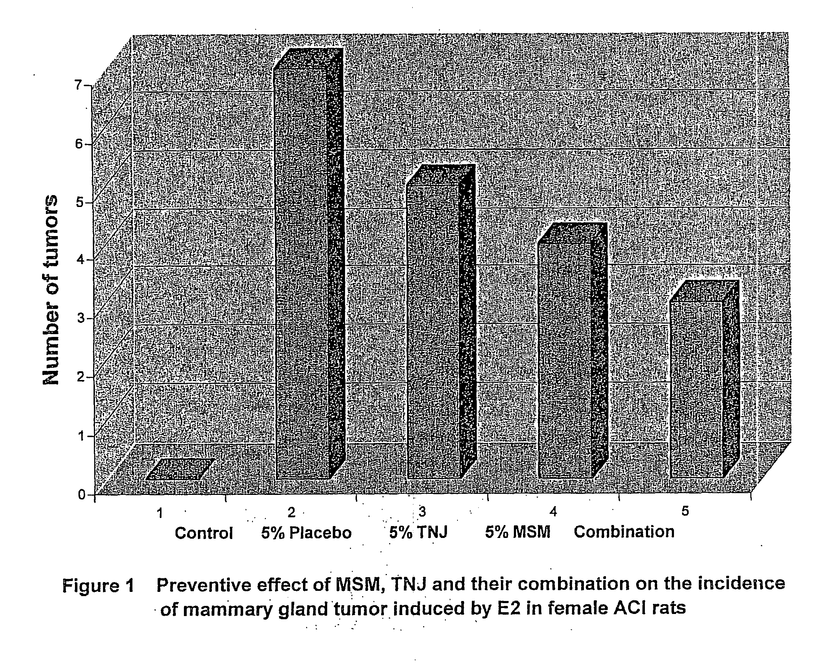

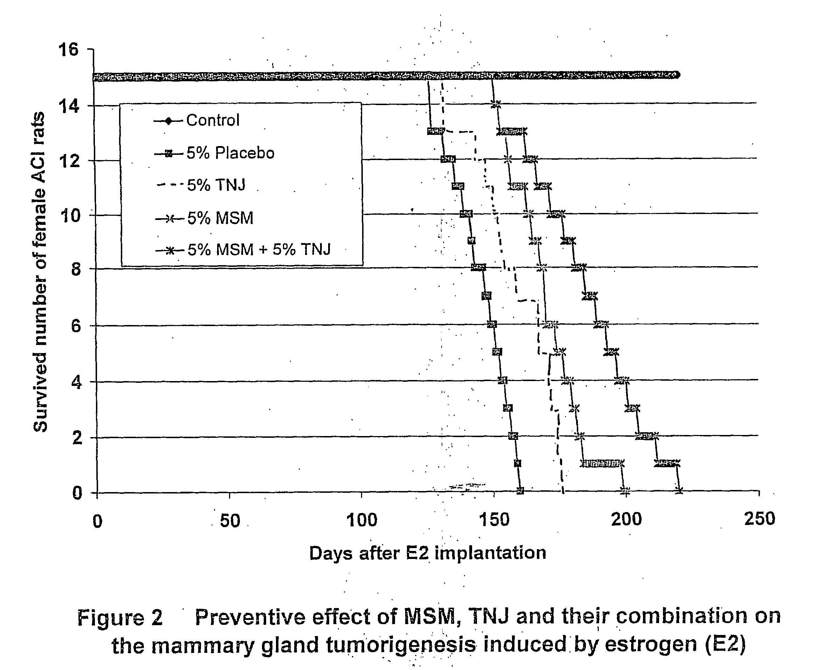

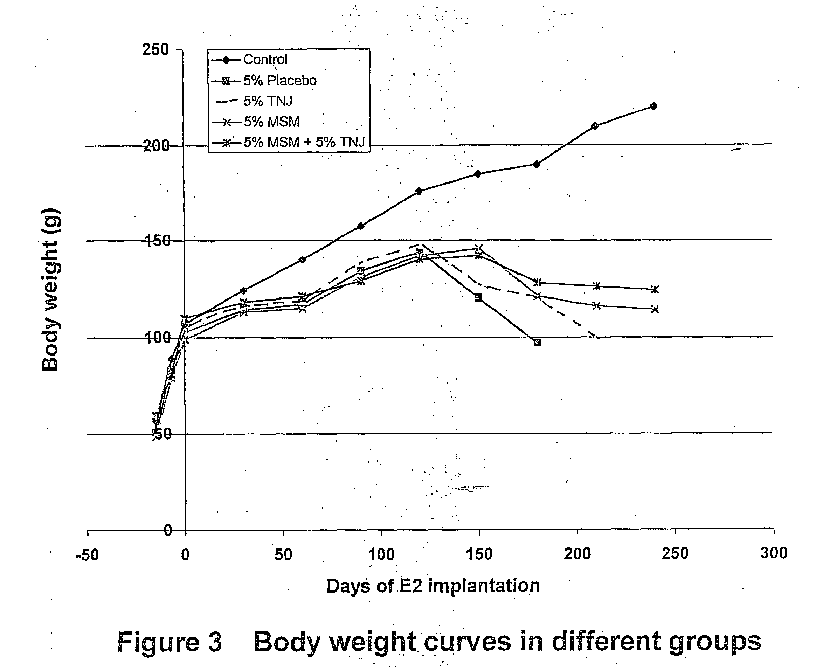

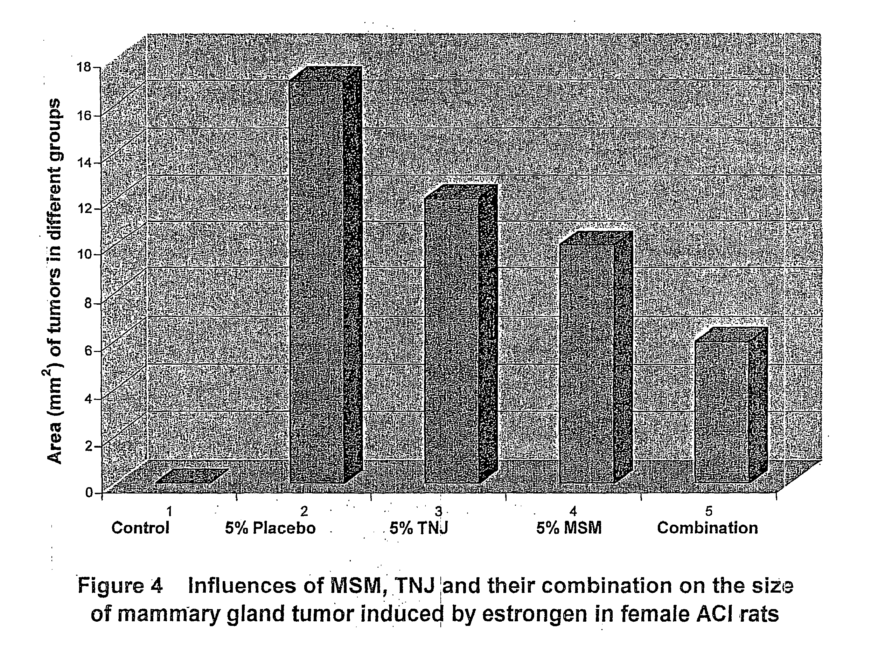

As seen in Figures 1-4, the animals in the placebo group had a significant body weight loss when compared to the cholesterol control group. The animals in the Morinda citrifolia group or MSM group had slight body weight loss. None of the rats that received the pellets composed of cholesterol exhibited mammary tumors. All rats with an E2 implant in the placebo group had mammary gland tumors. One hundred percent (100%) of the rats in this group had three to seven tumors. Seventy-one percent (71%) of the rats in the Morinda citrifolia group had two to five tumors. Fifty-seven percent (57%) of the rats in the MSM group had one to four tumors. Forty-three percent (43%) of the rats in the combination group had zero to three tumors.

The average tumor area in the placebo group, Morinda citrifolia group, MSM group, and combination group at 180 days after E2 implantation were 17, 12, 10 and 6 mm², respectively. The survival rates of the control, placebo, Morinda citrifolia, MSM and combination groups one hundred and sixty days after E2 implantation were 100%, 0%, 47%, 73%, and 87%, respectively. The survival rates of each group at one hundred eighty days after E2 implantation was 100%, 0%, 0%, 20% and 60%, respectively. At two hundred days, the survival rates were 100%, 0%, 0%, 0%, and 27%, respectively.

## Claims

1. A composition for inhibiting carcinogen-mediated conversion of mammary gland cells to melanomas, comprising *Morinda citrifolia* fruit juice, methyl sulfonyl methane and water, wherein the *Morinda citrifolia* fruit juice is present in an amount of 5 percent by weight and wherein the methyl sulfonyl methane is present in an amount of 5 percent by weight.

2. The composition of claim 1, wherein said water is present in an amount of 90 percent by composition weight.

## Patentansprüche

1. Zusammensetzung zur Hemmung der durch Krebserregung vermittelten Umwandlung von Brustdrüsenzellen in Melanome, umfassend *Morinda citrifolia* Fruchtsaft, Methylsulfonylmethan und Wasser, worin der *Morinda citrifolia* Fruchtsaft in einer Menge von 5 Gewichtsprozent vorhanden ist, und worin das Methylsulfonylmethan in einer Menge von 5 Gewichtsprozent vorhanden ist.

2. Zusammensetzung nach Anspruch 1, worin das Wasser in einer Menge von 90% des Zusammensetzungsgewichts vorhanden ist.

## Revendications

1. Composition destinée à inhiber la conversion induite par carcinogènes des cellules de glande mammaire en mélanomes, comprenant du jus de fruit de *Morinda citrifolia,* du méthylsulfonylméthane et de l'eau, dans laquelle le jus de fruit de *Morinda citrifolia* est présent dans une quantité de 5 pour cent en poids, et dans laquelle le méthylsulfonylméthane est présent dans une quantité de 5 pour cent en poids.

2. Composition selon la revendication 1, dans laquelle ladite eau est présente dans une quantité de 90 pour cent en poids de la composition.
